# EUROPEAN PATENT APPLICATION

(11) **EP 4 643 853 A1**
(43) Date of publication of application: **05.11.2025**
(21) Application number: 23731502.3
(22) Date of filing: 10.01.2023
(51) Int. Cl.: A61K 9/58, A61K 47/34, A61K 47/24, A61K 47/10, A61K 31/436, A61L 29/08

(54) **CORE-SHELL STRUCTURE MICELLAR MICROSPHERE, PREPARATION METHOD THEREFOR, AND USE THEREOF**

(30) Priority: 28.12.2022 CN 202211697363
(71) Applicant: Shanghai Bio-Heart Biological Technology Co., Ltd., Shanghai 201201 (CN)
(72) Inventor: WANG, Philip Li., Shanghai 201201 (CN); CAI, Tao., Shanghai 201201 (CN); WANG, Junyi., Shanghai 201201 (CN); LIU, Hanyang., Shanghai 201201 (CN); ZHANG, Chenzhao., Shanghai 201201 (CN)
(74) Representative: Jannig & Repkow Patentanwälte PartG mbB
(86) International application number: PCT/CN2023/071539
(87) International publication number: WO 2024/138798

(57) **Abstract**

The present invention belongs to the technical field of medicines, and specifically, relates to a core-shell micelle microsphere, and a preparation method therefor and use thereof. The core-shell micelle microsphere of the present invention has an outer layer shell composed of an amphiphilic lipid, an inner layer shell formed by a hydrophilic chain polymer, and a drug molecule inner core wrapped by a copolymer with a net structure. A drug may be effectively transported through the two-layer shell structure, such that a loss of the drug in a transportation process is reduced, and a utilization rate of the drug is improved. A slow release of the drug is realized by using a wrapped drug molecule as the inner core. The present invention also optimizes a core-shell material, and the amphiphilic phospholipid is selected as the outer layer shell, so as to improve biocompatibility and drug delivery efficiency; the hydrophilic chain polymer is selected as the inner layer shell, so as to effectively prevent a burst release of the drug; and an mPEG-PLGA block copolymer is selected to wrap the drug molecules, so as to further prevent the burst release of the drug, to realize a control of a drug release rate, and to meet a complex requirement in practical use.

## Description

### FIELD OF TECHNOLOGY

The present invention relates to the technical field of medicines, particularly to the field of composite materials of a coated catheter under IPC No. A61L29/12, more specifically to a core-shell micelle microsphere, and a preparation method therefor and use thereof.

### BACKGROUND

A microsphere preparation is a new dosage of a drug developed in recent years by dispersing the drug in a matrix material to form a spherical particle that may be administered by an injection or an interventional operation. After the administration, a skeleton of the microsphere is degraded and eroded, a coated drug molecule diffuses out, and thus an effect of a sustained release or a delayed release. At present, microsphere preparations with various structures such as a porous structure, a core-shell structure, a solid structure, and the like have existed.

In particular, an interventional treatment performed by an expandable balloon has been widely used in clinic today. In order to achieve a chemical drug treatment with high targeting, a surface of the expandable balloon is generally coated with a drug coating. However, both an injection dosage and the drug coating of an expandable balloon have a problem that a drug is lost in blood before reaching a target position, such that bioavailability of the drug is greatly reduced. Moreover, the microsphere preparation carries the drug mostly through an intermolecular force such as an electrostatic action, and thus the binding is not tight enough. Sometimes, the microsphere surface also contains a part of the drug. This causes a burst release, has a risk of causing an adverse reaction, also enables a release period of the drug to be too short, and may not play a long-acting therapeutic effect. Therefore, it becomes an urgent need in the field of current pharmaceutical preparations to develop a microsphere with a stable structure and a good sustained-release effect.

The prior art CN105106174B discloses a core-shell double-layer microsphere prepared from polyketal and a polylactic acid-glycolic acid copolymer. The microsphere may solve a problem of a burst release, but has a larger particle size, has a high requirement on injection, may not be suitable for a drug coating of an expandable balloon, has a lower drug release ratio, and thus causes low bioavailability of a drug.

### SUMMARY

Aiming at the defects of the prior art, the present invention aims to provide a core-shell micelle microsphere which has a high drug utilization rate, is simple and convenient to prepare, has a smaller particle size and a wide application range, and may realize a long-term sustained release of a drug.

In another aspect, the present invention further aims to provide a preparation method for the core-shell micelle microsphere, which has a mild condition and a simple and convenient process.

In another aspect, the present invention further aims to provide use of the core-shell micelle microsphere.

In order to achieve the above objectives, the present invention uses the following technical solutions:

A core-shell micelle microsphere, wherein a core of the core-shell micelle microsphere is a plurality of drug molecules wrapped by a copolymer, and a shell of the core-shell micelle microsphere is a chain polymer layer and a lipid layer from inside to outside in sequence; the copolymer is of a net structure; and the chain polymer layer is a net structure formed by the chain polymer through an intermolecular force.

Preferably, the core-shell micelle microsphere has a particle size of 1-10 µm.

Preferably, the lipid layer is composed of an amphiphilic lipid with a hydrophilic end facing into the shell and a lipophilic end facing out the shell.

Further preferably, the amphiphilic lipid is soybean phospholipid.

The core-shell micelle microsphere in the present invention has an outer layer composed of an amphiphilic lipid, which is arranged in a manner that a hydrophilic end faces into the shell and a lipophilic end faces out the shell. The hydrophilic end facing into the shell may be compatible with a hydrophilic inner layer to form a compact double-layer structure, so as to prevent a diffusion of an inner core drug molecule, and during a preparation, a double-layer spherical shell may be formed in a solvent by means of a similar polarity, such that a preparation process is simple and convenient. Since the lipophilic end faces out the shell, during transportation in blood, the lipophilic end has a large polarity difference with blood, may form a hydrophobic surface outside the shell, protects a drug molecule from being diffused in blood, reduces a drug loss in a transportation process, and improves bioavailability of a drug. Besides, when the microsphere contacts with a blood vessel wall, compatibility between the lipophilic end and a cell membrane is good, particularly phospholipid and the cell membrane have a similar composition and better compatibility, a lipid layer and the cell membrane are dissolved mutually to open a drug delivery channel, so as to realize a drug release and further improve a utilization rate of the drug. Particularly, when the core-shell micelle microsphere of the present invention is used as a drug coating of an expandable drug balloon, the microsphere is contacted with a vessel wall at a target position through an expansion of a balloon, a residence of the drug at a target position is further realized by a mutual dissolution of the lipid layer and the cell membrane, a targeting property is improved, damage to a healthy tissue is reduced, and an adverse reaction is reduced.

Preferably, the chain polymer is a hydrophilic chain polymer and comprises one or more of polyethylene glycol (PEG), hyaluronic acid, chitosan, iopromide, shellac, tannic acid, polylactide, and a polylactic acid-glycolic acid (PLGA) copolymer.

Preferably, a mass fraction of the drug molecule in the core-shell micelle microsphere is 30%-50%.

Preferably, a mass ratio of the lipid layer to the chain polymer layer is (1-5):1.

The present invention selects the hydrophilic chain polymer to form a net structure as an inner layer shell of the core-shell micelle microsphere, in addition to that the inner shell is compatible with a hydrophilic end of the amphiphilic lipid layer to form a double-layer spherical shell, when the microsphere is dissolved with a blood vessel wall, the chain polymer is degraded to release a drug, such that the drug is prevented from a burst release, and an effect of a sustained release or delayed release is realized. The inventor creatively discovers that a particle size and a morphology of the microsphere may be regulated and controlled by adjusting a weight ratio of the lipid to the hydrophilic chain polymer. The present invention specifically selects the weight ratio of the lipid to the hydrophilic chain polymer of (1-5):1, a core-shell micelle microsphere with a relatively regular shape and moderate size with a particle size less than or equal to 10+0.5 µm is obtained, such that the microsphere may be used as a drug coating of an expandable drug balloon, and an application range is expanded. Meanwhile, when the core-shell micelle microsphere of the present invention is used as a drug coating of an expandable drug balloon, the hydrophilic chain polymer absorbs water to enable the drug coating to be easily stripped from a surface of a balloon, and a defect of a low utilization rate of a drug caused by an excessive residue of the coating on the balloon is overcome.

Preferably, the copolymer is selected from one or more of an mPEG-PLGA block copolymer, a PEG-PLGA block copolymer, and a PEG-hyaluronic acid copolymer.

Preferably, a mass fraction of methoxy polyethylene glycol (mPEG) in the mPEG-PLGA block copolymer is 0.1-5.0%, further preferably, 1%.

Preferably, a number-average molecular weight of the mPEG in the mPEG-PLGA block copolymer is 550-2,000, and a number-average molecular weight of PLGA is 5,000-60,000.

The present invention specifically selects a net structure formed by a block copolymer consisting of the mPEG with a number-average molecular weight of 550-2,000 and the PLGA with a number-average molecular weight of 5000-60,000, a drug molecule is wrapped, a drug is released along with a degradation at a target position, such that a drug release rate is further adjusted. The inventor also surprisingly finds that when a proportion of the mPEG is greater, a drug release rate is faster; when the proportion of the mPEG is greater than 5 wt%, the drug release rate is faster and a burst release may even occur; when the proportion of the mPEG is 1 wt%, a drug may be quickly released in an early stage to improve a blood concentration, the drug release rate in a later stage is slowed down, and the drug may be continuously released for 90 days; and if the proportion of the mPEG is 0.1 wt%, the drug release rate of the microsphere is slower and almost linear, and a stable release with an almost constant rate is realized. Based on the creative discovery, the core-shell micelle microsphere of the present invention may be endowed with different drug release rate characteristics by adjusting the proportion of the mPEG in the mPEG-PLGA block copolymer, so as to meet various complex requirements in clinical practical use.

Another aspect of the present invention provides a preparation method for the core-shell micelle microsphere, comprising the following steps:
S1: dissolving a drug and the copolymer in an organic solvent A, uniformly mixing same, fully dissolving the mixture, and performing standing for 2-8 hours to obtain a mixed solution a;
S2: dissolving the lipid and the chain polymer in a solvent B, and uniformly mixing same to obtain a mixed solution b;
S3: adding the mixed solution a into the mixed solution b, and uniformly mixing same to obtain a core-shell micelle microsphere solution; and
S4: drying and desolventizing the core-shell micelle microsphere solution to obtain a core-shell micelle microsphere.

Preferably, the drug is one or more selected from rapamycin, a rapamycin derivative, paclitaxel, heparin, and hirudin.

Preferably, the drug is 20-80 parts by weight, the lipid is 10-70 parts by weight, the chain polymer is 5-60 parts by weight, and the copolymer is 5-60 parts by weight.

Preferably, the organic solvent A comprises one or more of ethanol, isopropanol, acetone, dichloromethane, trichloromethane, ethyl acetate, acetonitrile, butyl acetate, diethyl ether, and carbon tetrachloride.

Preferably, the solvent B comprises one or more of methanol, ethanol, isopropanol, dichloromethane, trichloromethane, ethyl acetate, acetonitrile, butyl acetate, diethyl ether, and carbon tetrachloride.

Preferably, the organic solvent A and the solvent B are both 10,000-25,000 parts by weight.

Another aspect of the present invention provides use of the core-shell micelle microsphere in a drug coating or injection for an expandable drug balloon.

Preferably, a preparation method for the drug coating comprises the following steps: ultrasonically atomizing the core-shell micelle microsphere solution to form a fogdrop, spraying the fogdrop onto a balloon surface of an expandable balloon catheter with nitrogen, during a spraying process, volatilizing a solvent, and adhering the core-shell micelle microsphere onto the balloon surface to form the drug coating.

Preferably, the drug coating has a thickness of 1-10 µm.

Compared with the prior art, the present invention has the following beneficial effects:
1. The core-shell micelle microsphere in the present invention has an outer layer composed of an amphiphilic lipid, so as to prevent a diffusion of an inner core drug molecule, forms a hydrophobic surface outside a shell, reduces a drug loss in a transportation process, and improves bioavailability of a drug.
2. The present invention selects the amphiphilic lipid, especially phospholipid, as an outer layer shell structure, which has an excellent compatibility with a cell membrane, is mutually dissolved with the cell membrane to realize a drug release, improves a utilization rate of a drug, meanwhile, may also realize a residence of the drug at a target position, improves a targeting property, and reduces an adverse reaction.
3. The present invention selects the hydrophilic chain polymer to form a net structure as an inner layer shell of the core-shell micelle microsphere, such that the drug is prevented from a burst release, and an effect of a sustained release or delayed release is realized.
4. The present invention may regulate and control a particle size and a morphology of the microsphere by adjusting a weight ratio of the lipid to the hydrophilic chain polymer. Especially, when the weight ratio of the lipid to the hydrophilic chain polymer of (1-5):1 is specifically selected, a microsphere with a relatively regular shape and a particle size less than or equal to 10+0.5 µm is obtained, and an application range of the core-shell micelle microsphere is expanded.
5. The present invention specifically selects the mPEG-PLGA block copolymer with a specific molecular weight to form a net structure, which wraps a drug molecule and further realizes a sustained release of the drug.
6. The present invention also creatively discovers a relationship between a proportion of the mPEG in the mPEG-PLGA block copolymer and a drug release rate of the obtained core-shell micelle microsphere. The microsphere may endowed with different drug release rate characteristics by adjusting the proportion of the mPEG in the mPEG-PLGA block copolymer, so as to meet various complex requirements in clinical practical use.

### BRIEF DESCRIPTION OF THE DRAWINGS

FIG. 1 is a scanning electron micrograph of the core-shell micelle microsphere of example 2;
FIG. 2 is a morphology photograph of the core-shell micelle microsphere of example 2 under an optical microscope;
FIG. 3 is a morphology photograph of the core-shell micelle microsphere of comparative example 1 under an optical microscope;
FIG. 4 is a schematic diagram of a drug molecule (green) wrapped by an mPEG-PLGA block copolymer (black);
FIG. 5 is a cross-section structural diagram of the core-shell micelle microsphere of the present invention;
FIG. 6 shows curve graphs showing changes of drug release rates of the core-shell micelle microspheres prepared from the mPEG-PLGA block copolymers with mPEG mass fractions of 0.1% (example 1), 1% (example 2), and 5% (example 3) with time; and
FIG. 7 shows a curve graph showing a change of a drug release rate of the core-shell micelle microsphere prepared from the mPEG-PLGA block copolymer with an mPEG mass fraction of 9% (comparative example 2) with time.

Reference numerals: 1, lipid layer, 2, chain polymer layer, 3, drug molecule, and 4, copolymer with net structure.

### DESCRIPTION OF THE EMBODIMENTS

The part in the examples refers to a part by weight.

### Example 1

The present example provided a core-shell micelle microsphere, wherein a core of the core-shell micelle microsphere was a plurality of drug molecules wrapped by a copolymer of a net structure, and a shell of the core-shell micelle microsphere was a net structure formed by a chain polymer and a lipid layer from inside to outside in sequence.

The lipid layer was composed of soybean phospholipid, and a hydrophilic end of the soybean phospholipid faced into the shell and a lipophilic end faced out the shell.

The chain polymer was PEG.

A mass ratio of the lipid layer to the net structure formed by the chain polymer was 3:2.

The drug molecule was a rapamycin molecule.

A mass fraction of the drug molecule in the core-shell micelle microsphere was 40%.

The copolymer was an mPEG-PLGA block copolymer, wherein a molecular weight of an mPEG was 550, a molecular weight of PLGA was 60,000, a mass fraction of the mPEG was 0.1%, and the mPEG-PLGA block copolymer was purchased from EVONIK (Shanghai).

The present example further provided a preparation method for the core-shell micelle microsphere, comprising the following steps:
S1: 40 parts of the rapamycin and 60 parts of the mPEG-PLGA block copolymer were dissolved in 20,000 parts of acetone, the materials were uniformly mixed and fully dissolved, and standing was performed for 6 hours to obtain a mixed solution a;
S2: 60 parts of the soybean phospholipid and 40 parts of the PEG were dissolved in 20,000 parts of ethyl acetate, and the materials were uniformly mixed to obtain a mixed solution b;
S3: the mixed solution a was added into the mixed solution b to be uniformly mixed to obtain a core-shell micelle microsphere solution; and
S4: the core-shell micelle microsphere solution was spray-dried and desolventized to obtain a core-shell micelle microsphere.

The present example further provided use of the core-shell micelle microsphere in a drug coating or injection for an expandable drug balloon.

Preferably, a preparation method for the drug coating comprised the following steps: the core-shell micelle microsphere solution was ultrasonically atomized to form a fogdrop, the fogdrop was sprayed onto a balloon surface of an expandable balloon catheter with nitrogen, during a spraying process, a solvent was volatilized, and the core-shell micelle microsphere was adhered onto the balloon surface to form the drug coating.

The drug coating had a thickness of 10 µm.

### Example 2

The present example provided a core-shell micelle microsphere, and a preparation method therefor and use thereof. The embodiment was the same as that in example 1, except that the mass fraction of the mPEG in the mPEG-PLGA block copolymer was 1%, and the mPEG-PLGA block copolymer was purchased from EVONIK (Shanghai).

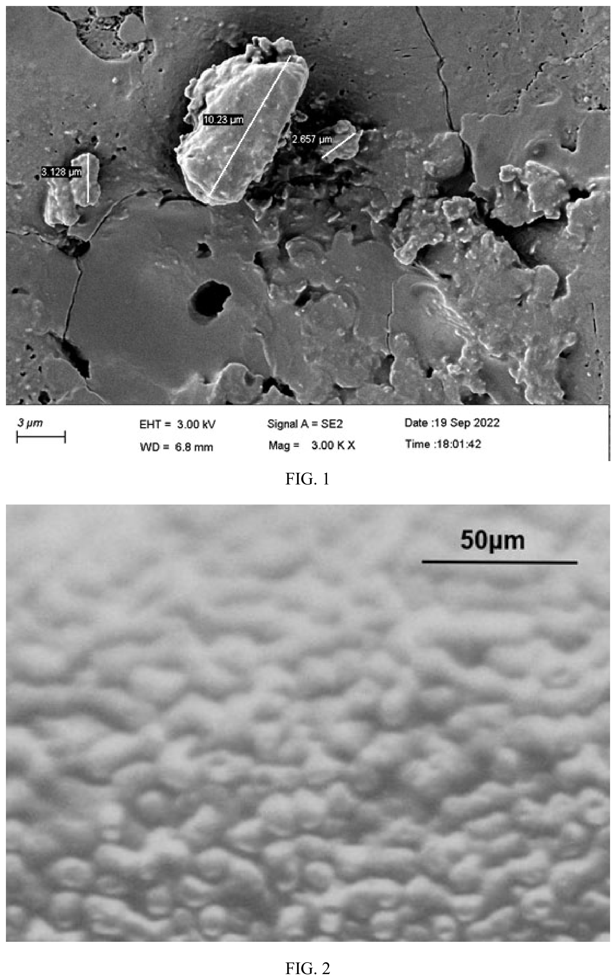

The core-shell micelle microsphere of the present example had a particle size of 2.5-10.5 µm. A scanning electron micrograph was shown in FIG. 1 and a morphology photograph under an optical microscope was shown in FIG. 2.

### Example 3

The present example provided a core-shell micelle microsphere, and a preparation method therefor and use thereof. The embodiment was the same as that in example 1, except that the mass fraction of the mPEG in the mPEG-PLGA block copolymer was 5%, and the mPEG-PLGA block copolymer was purchased from EVONIK (Shanghai).

### Comparative example 1

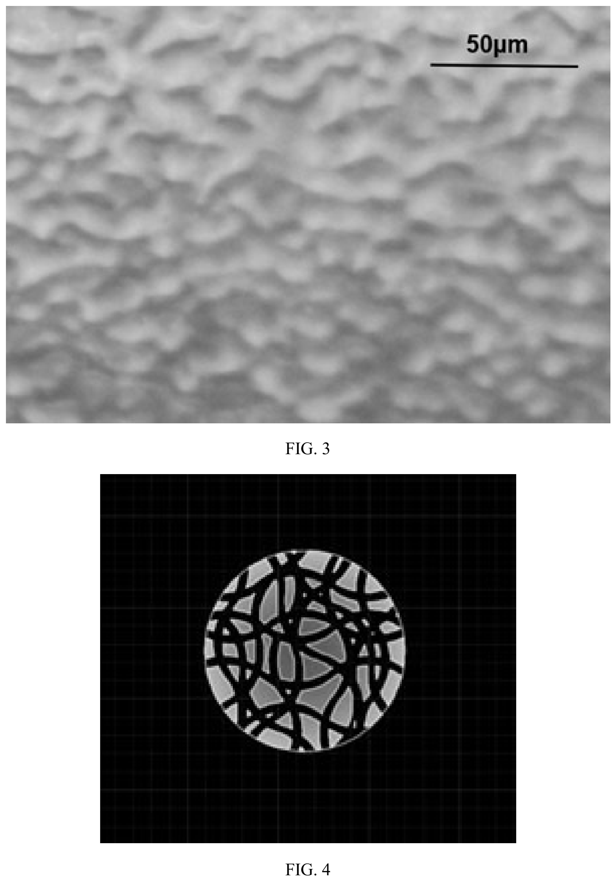

The present example provided a core-shell micelle microsphere, and a preparation method therefor and use thereof. The embodiment was the same as that in example 2, except that the mass ratio of the lipid layer to the net structure formed by the chain polymer was 1: 3. A morphology photograph under an optical microscope was shown in FIG. 3.

### Comparative example 2

The present example provided a core-shell micelle microsphere, and a preparation method therefor and use thereof. The embodiment was the same as that in example 2, except that the mass fraction of the mPEG in the mPEG-PLGA block copolymer was 9%, and the mPEG-PLGA block copolymer was purchased from Jinan Jufukai Biotechnology Co., Ltd.

### Performance test

Drug release test: in order to verify a sustained-release effect of the core-shell micelle microspheres prepared from the mPEG-PLGA block copolymers with different mPEG mass fractions, an animal experiment was used for testing. In this experiment, an animal model of mini-pigs was used, the drug coatings prepared in examples 1-3 and comparative example 2 were used in mini-pigs respectively, and time nodes were set at 0 (instant) day, 1 day, 7 days, 14 days, 30 days, 60 days, and 90 days. A pig blood vessel was taken and subjected to a drug detection.

Experimental subject: the model of mini-pigs, weighed about 25-45 kg.

Experimental product: the drug coatings in examples 1-3 and comparative example 2 with a drug content of 1-10 µg/mm².

### Experimental solution

(1) 7 pigs were selected to each group of the animal model, corresponding to the time nodes of 0 (instant) day, 1 day, 7 days, 14 days, 30 days, 60 days, and 90 days respectively. Cardiac vessel RCA, LCX, and LAD of each group of the pig animal model were respectively implanted into a balloon of an expandable balloon catheter sprayed with the drug coatings in examples 1-3 and comparative example 2.
(2) The pigs were sacrificed at the time nodes of 0 (instant) day, 1 day, 7 days, 14 days, 30 days, 60 days, and 90 days, and the blood vessel was taken for detecting the drug content.

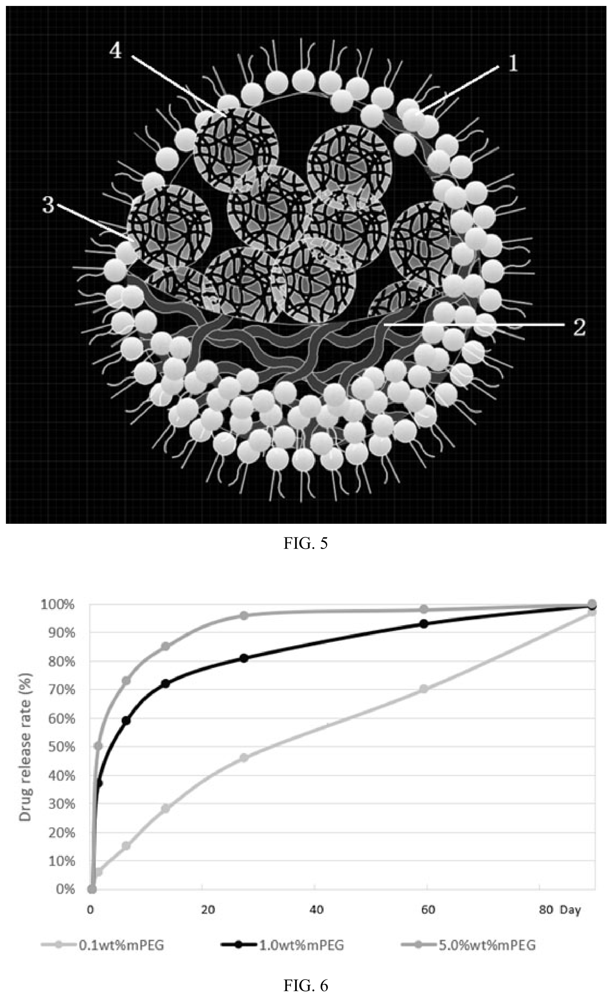

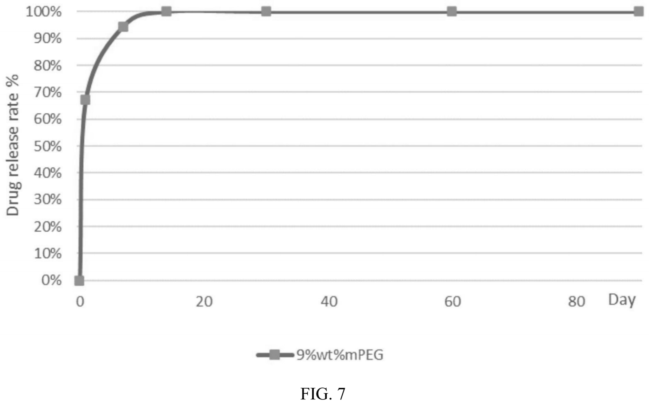

Experimental result: drug release curves were plotted according to the measured drug content (shown in FIGs. 6 and 7), and the drug release ratio on a y coordinate was a ratio of the content of the drug on a blood vessel wall to the content of the drug on the balloon surface before the implantation.

## Claims

1. A core-shell micelle microsphere, **characterized in that** a core of the core-shell micelle microsphere is a plurality of drug molecules wrapped by a copolymer, and a shell of the core-shell micelle microsphere is a chain polymer layer and a lipid layer from inside to outside in sequence; the copolymer is of a net structure; and the chain polymer layer is a net structure formed by the chain polymer through an intermolecular force.

2. The core-shell micelle microsphere according to claim 1, **characterized in that** the core-shell micelle microsphere has a particle size of 1-10 µm.

3. The core-shell micelle microsphere according to claim 1, **characterized in that** the lipid layer is composed of an amphiphilic lipid with a hydrophilic end facing into the shell and a lipophilic end facing out the shell.

4. The core-shell micelle microsphere according to claim 1, **characterized in that** the chain polymer is a hydrophilic chain polymer and comprises one or more of PEG, hyaluronic acid, chitosan, iopromide, shellac, tannic acid, polylactide, and PLGA.

5. The core-shell micelle microsphere according to claim 1, **characterized in that** the copolymer is selected from one or more of an mPEG-PLGA block copolymer, a PEG-PLGA block copolymer, and a PEG-hyaluronic acid copolymer.

6. The core-shell micelle microsphere according to claim 5, **characterized in that** a mass fraction of mPEG in the mPEG-PLGA block copolymer is 0.1-5.0%.

7. The core-shell micelle microsphere according to claim 1, **characterized in that** a mass ratio of the lipid layer to the chain polymer layer is (1-5): 1.

8. A preparation method for the core-shell micelle microsphere according to any one of claims 1-7, comprising the following steps:
S1: dissolving a drug and the copolymer in an organic solvent A, uniformly mixing same, fully dissolving the mixture, and performing standing for 2-8 hours to obtain a mixed solution a;
S2: dissolving the lipid and the chain polymer in a solvent B, and uniformly mixing same to obtain a mixed solution b;
S3: adding the mixed solution a into the mixed solution b, and uniformly mixing same to obtain a core-shell micelle microsphere solution; and
S4: drying and desolventizing the core-shell micelle microsphere solution to obtain a core-shell micelle microsphere.

9. Use of the core-shell micelle microsphere according to any one of claims 1-7 or the core-shell micelle microsphere obtained by the preparation method according to claim 8 in a drug coating or injection for an expandable drug balloon.

10. Use of the core-shell micelle microsphere according to claim 9, **characterized in that** the drug coating has a thickness of 1-10 µm.
